# EUROPEAN PATENT APPLICATION

(11) **EP 3 659 515 A1**
(43) Date of publication of application: **03.06.2020**
(21) Application number: 18209297.3
(22) Date of filing: 29.11.2018
(51) Int. Cl.: A61B 8/00

(54) **IMAGING SYSTEM COMPRISING AN ULTRASOUND TRANSDUCER ARRAY AND SKIN CONTACT ELECTRODES, AND CORRESPONDING IMAGING METHOD**

(71) Applicant: Koninklijke Philips N.V., 5656 AG Eindhoven (NL)
(72) Inventor: GIJSBERS, Gerardus Henricus Maria, 5656 AE Eindhoven (NL); JOHNSON, Mark Thomas, 5656 AE Eindhoven (NL); DE WILD, Nico Maris Adriaan, 5656 AE Eindhoven (NL)
(74) Representative: de Haan, Poul Erik

(57) **Abstract**

An ultrasound imaging system is provided in which skin contact electrodes (210) are formed on the same substrate as an array of transducer elements. Skin impedance measurement is performed using the skin contact electrodes and the skin impedance is analyzed over time to determine a pain or discomfort level of the subject. This information may be used to adapt the imaging process (e.g. the position of the imaging system on the skin of a subject and the pressure applied) thereby to reduce discomfort.

## Description

### FIELD OF THE INVENTION

The present invention relates to ultrasound transducers for use in an ultrasound imaging system.

### BACKGROUND OF THE INVENTION

Several recent studies document the decline in physical examination skills among physicians. Reasons postulated for this shift are improvements in technology, time constraints, and uncertainty that stems from a lack of confidence in physical examination skills.

Standard equipment (e.g. stethoscope and tuning fork) and methods for physical examination have been applied since the early 19th century. This equipment requires skill and practice to be used and it is shown that the results obtained by this standard equipment are far from objective and reproducible.. Thus, this skill and practice is declining as new imaging technology tends to to decrease the priority of physical examination. However, it is likely that some form of initial patient-physician interaction will always be required. Technology integration, such as hand-held ultrasound and video examination, is required to keep physical examination up-to-date with modern medicine

One physical examination of particular interest is physical examination of the heart (i.e. a cardiovascular examination). The cardiovascular examination aims to pick up on any cardiovascular pathology that may be causing a patient's symptoms, such as chest pain, breathlessness, or heart failure. However, this is a complex examination. Although still widely used, auscultation by means of a stethoscope yields low accuracy and reliability.

Key observations made during the examination of the heart are the heart rate, the size of the heart (as an indication for enlargement of the left ventricle) and valve function and blood flow. This is for example observed via auscultation of the heart at four standard positions, related to the different heart valves; mitral valve, aortic valve, tricuspid valve, pulmonary valve. Heart sounds and murmurs give indications for valve defects, volume overload, pressure overload and hypertrophy.

An echocardiogram is an ultrasound test that can better evaluate the structures and pathologies of the heart, as well as the direction of blood flow within it, than auscultation. Technicians specially trained in echocardiography produce the images and videos, often using a special probe or transducer that is placed in various places on the chest wall, to view the heart from different directions. Cardiologists, or heart specialists, are trained to evaluate these images to assess heart function and provide a report of the results. Information from the echocardiogram may give the following information:
The heart size: weakened or damaged heart valves, high blood pressure, or other diseases can cause the chambers of the heart to enlarge or the walls of the heart to be abnormally thickened;
The pumping strength: an echocardiogram can help to determine the heart pumping strength. Specific measurements may include the percentage of blood that is pumped out of a filled ventricle with each heartbeat (ejection fraction) or the volume of blood pumped by the heart in one minute (cardiac output);
Damage to the heart muscle: during an echocardiogram, it can be determined whether all parts of the heart wall are contributing normally to the heart's pumping activity. Parts that move weakly may have been damaged during a heart attack or be receiving too little oxygen. This may indicate coronary artery disease or various other conditions;
Valve problems: an echocardiogram shows how the heart valves move as the heart beats. It can then be determined if the valves open wide enough for adequate blood flow (i.e. no stenosis) or close fully to prevent blood leakage (i.e. no regurgitation);
Heart defects: many heart defects can be detected with an echocardiogram, including problems with the heart chambers, abnormal connections between the heart and major blood vessels, and complex heart defects that are present at birth. Echocardiograms can even be used to monitor a baby's heart development before birth.

Additionally, with advanced imaging techniques, wall thickness, wall kinetics and flow patterns may be assessed.

Ultrasonic transducers used for medical imaging have numerous characteristics that lead to the production of high quality diagnostic images, and it is expected that ultrasound imaging may gradually replace auscultation in the cardiovascular examination.

Traditionally, piezoelectric materials have been used for ultrasonic transducers. Examples are lead zirconate titanate (PZT) and polyvinylidene difluoride (PVDF) materials, with PZT being particularly popular as the material of choice. Single crystal piezoelectric materials are used to achieve high piezoelectric and electro-mechanical coupling constants for high performance transducers.

Recent developments have led to the prospect that medical ultrasound transducers can be batch manufactured by semiconductor processes. Desirably these processes should be the same ones used to produce the application specific integrated circuits (ASICs) needed by an ultrasound probe such as a CMOS process, particularly for 3D ultrasound. These developments have produced micro machined ultrasonic transducers or MUTs, the preferred form being the capacitive MUT (CMUT). CMUT transducers are tiny diaphragm-like devices with electrodes that convert the sound vibration of a received ultrasound signal into a modulated capacitance.

CMUT transducers in particular are able to function over a broad bandwidth, enable high resolution and high sensitivity imaging, and produce a large pressure output so that a large depth of field of acoustic signals can be received at ultrasonic frequencies.

For transmission, the capacitive charge applied to the electrodes is modulated to vibrate/move the diaphragm of the device and thereby transmit an ultrasound wave. Since these diaphragms are manufactured by semiconductor processes the devices generally can have dimensions in the 10-500 micrometer range, with the diaphragm diameter for instance being selected to match the diaphragm diameter to the desired resonance frequency (range) of the diaphragm, with spacing between the individual diaphragms less than a few micrometers.

Many such individual CMUT cells can be connected together and operated in unison as a single transducer element. For example, four to sixteen CMUT cells can be coupled together to function in unison as a single transducer element. A typical 2D transducer array can have 2000-10000 CMUT transducer elements or cells by way of example.

The manufacture of CMUT transducer-based ultrasound systems is therefore more cost-effective compared to PZT-based systems. Moreover, due to the materials used in such semiconductor processes, the CMUT transducers exhibit much improved acoustic impedance matching to water and biological tissue, which obviates the need for (multiple) matching layers and yields an improved effective bandwidth.

To enable imaging of a large area, it is known to make use of an ultrasound probe which is moved over the skin of the subject being imaged. However, the process of pressing and moving the ultrasound probe over the skin can cause discomfort for the subject. It would be an advantage if a medical practitioner could have an objective measure of the discomfort suffered by the subject, so that the imaging procedure using the ultrasound probe could be adapted. However, there is no system available for providing this information as part of an imaging procedure.

### SUMMARY OF THE INVENTION

The invention is defined by the claims.

According to examples in accordance with an aspect of the invention, there is provided an imaging system comprising:
a sensor head for application to an external surface of a body for imaging tissue beneath the surface, wherein the sensor head comprises:
   an ultrasound transducer array; and
   at least one pair of skin contact electrodes; and
a controller, which is adapted to:
   control the ultrasound transducer array to generate ultrasound image data;
   perform skin impedance measurement using the skin contact electrodes; and
   analyze the skin impedance over time to determine a pain or discomfort level of the subject.

This imaging system performs ultrasound imaging, and additionally uses integrated electrodes to obtain skin impedance measurements. From the skin impedance measurements, it is possible to assess a comfort or pain level of the subject being imaged (for example while the sensor head is moved over the skin of the subject) and thereby adapt the pressure on the skin to reduce discomfort.

Thus, adding electrodes to the ultrasound probe allows for assessment of pain or stress with a single, cost effective system. The skin contact electrodes can easily be adapted to have multiple large (large compared to size of the transducer cells) electrodes integrated with the transducer. A pair of electrodes on a transducer can be easily incorporated in the transducer production process. The electrodes are brought into contact with the skin when the transducer is placed on the skin.

The controller may be implemented partially in the sensor head and partially in a remote control unit which is connected by a cable or else wirelessly to the sensor head. The sensor head typically comprises an ASIC which performs at least some of the controller functionality, in particular controlling the transmit and receive function of the transducers, or pre-processing electrode signals, but the remote control unit may perform the signal analysis. Thus, the term "controller" is intended to include the option of multiple units which together perform the set of functions as defined.

The skin conductivity measurements may for example be recorded and interpreted through a PC, tablet or smartphone device.

The controller may be adapted to determine a phasic skin response. This provides a measure of the pain or discomfort, in particular by separating the phasic skin response from the tonic skin response.

The controller may be further adapted to perform ECG measurements using the skin contact electrodes.

In this way, as part of a physical examination, it is possible to measure different skin related signals indicative of certain medical conditions, e.g. pain or stress sensation based on the skin impedance measurement (in particular the phasic response, otherwise known as the Galvanic Skin Response, GSR) as well as an ECG trace. These two types of signal may be obtained sequentially or simultaneously. A separate ECG system is known to be included in a physical examination, especially to support assessment of heart failure and to diagnose cardiac arrhythmia, but it requires additional 2, 3 or 12 lead ECG systems that are usually not readily available.

The ultrasound transducer array preferably comprises a capacitive micromachined ultrasound transducer, CMUT, array.

Each transducer of the CMUT array may comprise:
a first electrode connected to the substrate;
a flexible membrane, wherein the flexible membrane is at least partially spatially separated from the first electrode; and
a second electrode connected to the flexible membrane,

This defines an electrode layout for a collapsible CMUT cell.

The controller may comprise drive electronics having a first output for delivering a DC bias voltage to first electrodes of the CMUT transducers and a second output for delivering an AC drive signal to the second electrodes of the CMUT transducers.

The drive electronics preferably comprises a capacitance sensing circuit.

The capacitance sensing circuit is used to measure a variation in the cell's capacitance as the response of the cell to an incident ultrasound stimulus and thereby enable ultrasound imaging.

The skin contact electrodes are preferably formed from the same layer as the second electrodes. Thus, a fully integrated solution is provided which enables skin impedance measurement and optionally also ECG measurement with little or no additional overhead.

The skin contact electrodes are preferably larger in area than the individual CMUT transducers. Thus, the different electrode areas may be optimized for their different functions.

The skin contact electrodes may for example have a maximum linear dimension in the range 1mm to 1cm and individual CMUT transducer cells for example have a maximum linear dimension in the range 0.01 to 0.5 mm. A single transducer may be formed of multiple such cells connected electrically in parallel.

The skin contact electrodes may each comprise a connected set of second electrodes of a sub-array of the CMUT transducers. In this way, the general array structure does not need to be modified to form the skin contact electrodes. Instead, areas of CMUT cells are redefined as skin contact electrodes. They are for example at edge locations around the CMUT array.

The controller may comprise an application specific integrated circuit, ASIC, wherein the CMUT transducers are integrated with the ASIC. The ASIC for example comprises a signal amplifier for the signals received by the skin contact electrodes, as well as the control electronics for ultrasound imaging. Thus the signal processing of the skin contact electrode signals may be integrated into the structure of the CMUT array, as well as the electrode structure.

The ultrasound probe may be connected to a control system be cable, or else the system may further comprise a wireless transmitter for transmitting ultrasound data and data from the skin contact electrodes to a remote device for display and/or processing. This enables remote processing and/or display at a more convenient location than at or in a probe.

The system may be controllable independently as:
an ultrasound probe;
a skin impedance analysis system, and
an ECG recordal system

Thus, a multifunction device is provided.

The invention also provides an imaging method comprising:
applying a sensor head to an external surface of a body for imaging tissue beneath the surface, wherein the sensor head comprises an ultrasound transducer array and least one pair of skin contact electrodes formed on the substrate;
controlling the transducer array to generate ultrasound image data;
performing skin impedance measurement using the skin contact electrodes; and
analyzing the skin impedance over time to determine a pain or discomfort level of the subject,
and optionally performing ECG measurements using the skin contact electrodes.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 schematically depicts a typical CMUT cell of an ultrasound system operable in a collapsed mode;
Figure 2a, 2b, 3a and 3b depict operating principles of such a CMUT cell;
Figure 4 shows an array of ultrasound transducer cells;
Figure 5 shows a first way to integrate skin contact electrodes with an array of ultrasound transducer cells;
Figures 6a and 6a show a transducer region and a skin contact electrode region, respectively, of the arrangement of Figure 5;
Figure 7 shows a second way to integrate skin contact electrodes with the array of ultrasound transducer cells;
Figure 8 shows an ultrasound imaging system; and
Figure 9 shows an ultrasound imaging method.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides an ultrasound imaging system in which skin contact electrodes are formed on the same substrate an array of transducer elements. Skin impedance measurement is performed using the skin contact electrodes and the skin impedance is analyzed over time to determine a pain or discomfort level of the subject. This information may be used to adapt the imaging process (e.g. the position of the imaging system on the skin of a subject and the pressure applied) thereby to reduce discomfort.

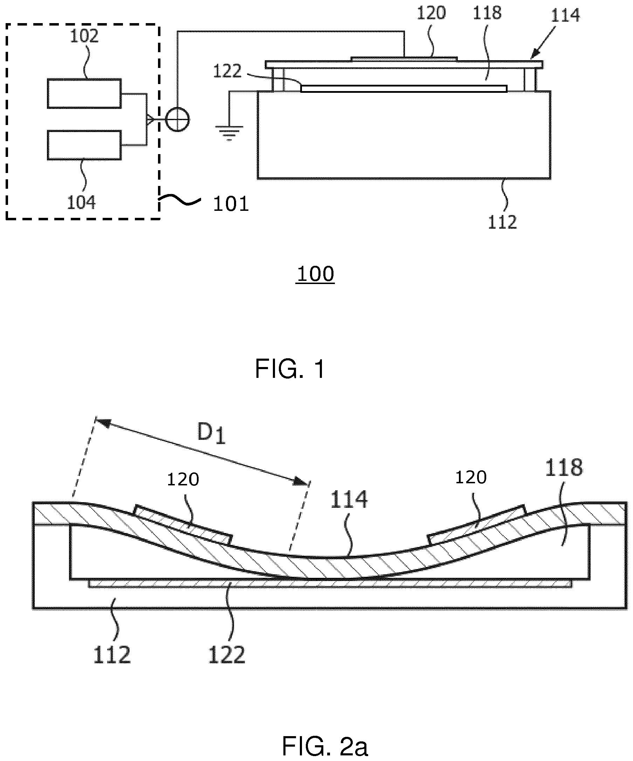

Figure 1 shows a known design of CMUT cell 100 for use in an ultrasound system. The CMUT cell 100 comprises a flexible membrane or diaphragm 114 suspended above a silicon substrate 112 with a gap or cavity 118 between them. A first electrode 122 is located on the floor of the cell on the upper surface of the substrate 112 in this example. A second electrode 120 is located on the diaphragm 114 and moves with the diaphragm. In the example shown, the two electrodes are circular.

A dielectric (not shown) is provided on the substrate 112 and underneath the top (second) electrode 120. These two dielectrics may be equal in composition and thickness, but may be also asymmetric (different materials and thicknesses).

The membrane layer 114 is fixed relative to the top face of the substrate layer 112 and configured and dimensioned so as to define a spherical or cylindrical cavity 118 between the membrane layer 114 and the substrate layer 112.

Other realizations of the electrode 120 design can be considered, such as electrode 120 may be embedded in the membrane 114 or it may be deposited on the membrane 114 as an additional layer. In this example, the first electrode 122 is circularly configured and embedded in the substrate layer 112 by way of non-limiting example. Other suitable arrangements are possible, such as other electrode shapes and other locations of the first electrode 122. The first electrode may be directly exposed to the gap 118 or separated from the gap 118 by an electrically insulating layer or film to prevent a short-circuit between the second electrode 120 and the first electrode 122.

In Figure 1 the first electrode 122 is grounded by way of non-limiting example. Other arrangements, e.g. a grounded second electrode 120 or both second electrode 120 and first electrode 122 floating are of course equally feasible.

The cell 100 and its gap 118 may have alternative geometries. For example, cavity 118 could have a rectangular or square cross-section, a hexagonal cross-section, an elliptical cross-section, or an irregular cross-section.

In this description, a largest linear dimension is intended to a dimension in the plane parallel to the substrate, so for a circular cell, the diameter of the CMUT cell 100 is taken to be the largest linear dimension of the cell.

In Figure 1, the diameter of the cylindrical cavity 118 is larger than the diameter of the circularly configured electrode plate 122. Electrode 120 may have the same outer diameter as the circularly configured electrode plate 122, although such conformance is not required and Figure 1 shows a larger electrode plate 122.

The electrodes of the CMUT cell 1 00 provide the capacitive plates of the device and the gap 118 is the dielectric between the plates of the capacitor. When the diaphragm vibrates, the changing dimension of the dielectric gap between the plates provides a changing capacitance which is sensed as the response of the CMUT cell 100 to a received acoustic echo.

The spacing between the electrodes is controlled by applying a static voltage, e.g. a DC bias voltage, to the electrodes with a voltage supply 101. The voltage supply 101 may optionally comprise separate stages 102, 104 for providing the DC and AC or stimulus components respectively of the drive voltage of the CMUT cells 100, e.g. in transmission mode. The first stage 102 may be adapted to generate the static (DC) voltage component and the second stage 104 may be adapted to generate an alternating variable drive or stimulus voltage component having a set alternating frequency, which signal typically is the difference between the overall drive voltage and the aforementioned static component thereof.

The static or bias component of the applied drive voltage preferably meets or exceeds the threshold voltage for forcing the CMUT cell 100 into its collapsed state. This has the advantage that the first stage 102 may include relatively large capacitors, e.g. smoothing capacitors, in order to generate a particularly low-noise static component of the overall voltage, which static component typically dominates the overall voltage such that the noise characteristics of the overall voltage signal will be dominated by the noise characteristics of this static component.

Other suitable embodiments of the voltage source supply 101 should be apparent, such as for instance an embodiment in which the voltage source supply 101 contains three discrete stages including a first stage for generating the static DC component of the CMUT drive voltage, a second stage for generating the variable but DC component of the drive voltage and a third stage for generating the frequency modulation or stimulus component of the signal, e.g. a pulse circuit or the like. It is summarized that the voltage source supply 101 may be implemented in any suitable manner.

It is known that by applying a static voltage above a certain threshold, the CMUT cell 100 is forced into a collapsed state in which the membrane 114 collapses onto the substrate 112. This threshold value may depend on the exact design of the CMUT cell 100 and is defined as the DC bias voltage, known as the collapse voltage, at which the membrane 114 sticks to (contacts) the cell floor through the force due to the electric field between the electrodes. The amount (area) of contact between the membrane 114 and the substrate 112 is dependent on the applied bias voltage.

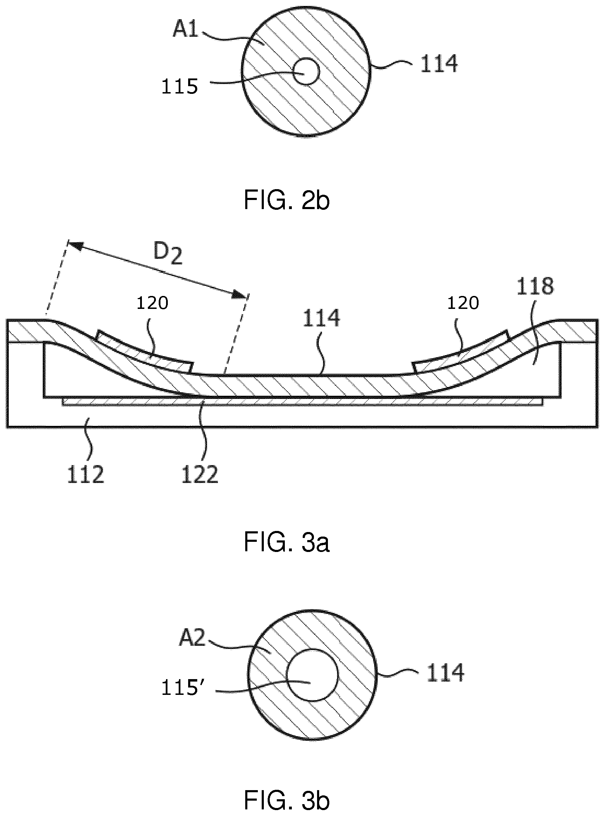

Increasing the contact area between the membrane 114 and the substrate 112 increases the resonant frequency of the membrane 114, as will be explained in more detail with the aid of Figure 2a and Figure 3a.

The frequency response of a collapsed mode CMUT cell 100 may be varied by adjusting the DC bias voltage applied to the CMUT electrodes after collapse. As a result, the resonant frequency of the CMUT cell increases as a higher DC bias voltage is applied to the electrodes.

The principles behind this phenomenon are illustrated in Figure 2a, 2b, 3a and 3b. The cross-sectional views of Figure 2a and 3a illustrate this one-dimensionally by the distances D1 and D2 between the outer support of the membrane 114 and the point where the membrane begins to touch the floor of the cavity 118 in each illustration. It can be seen that the distance D1 is a relatively long distance in Figure 2a when a relatively low bias voltage is applied, whereas the distance D2 in Figure 3a is a much shorter distance due to a higher bias voltage being applied. These distances can be compared to long and short strings which are held by the ends and then plucked. The long, relaxed string will vibrate at a much lower frequency when plucked than will the shorter, tighter string. Analogously, the resonant frequency of the CMUT cell in Figure 2a will be lower than the resonant frequency of the CMUT cell in Figure 3a which is subject to the higher bias voltage.

The phenomenon can also be appreciated from the two-dimensional illustrations of Figure 2b and 3b, which vary as a function of the effective operating area of the CMUT membrane. When the membrane 114 just touches the floor of the CMUT cell as shown in Figure 2a, the effective vibrating area A1 of the non-contacting (free vibrating) portion of the cell membrane 114 is large as shown in Figure 2b. The small area 115 in the center represents the center contact region of the membrane. The large area membrane will vibrate at a relatively low frequency. This area 115 is an area of the membrane 114 which is collapsed to the floor of the CMUT cell. When the membrane is pulled into deeper collapse by a higher bias voltage as in Figure 3a, the larger central contact area 115' results in a smaller free vibrating area A2 as shown in Figure 3b. This lesser area A2 will vibrate at a higher frequency than the larger A1 area. Thus, as the DC bias voltage is decreased the frequency response of the collapsed CMUT cell decreases, and when the DC bias voltage increases the frequency response of the collapsed CMUT cell increases.

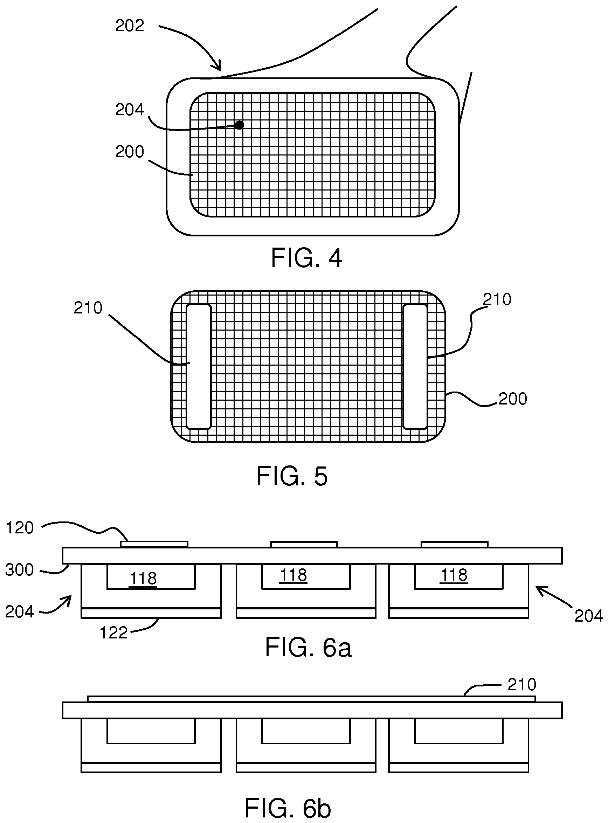

Figure 4 shows an imaging surface 200 of an ultrasound probe 202 and shows that the imaging surface comprises an array of CMUT cells 204, each having a structure as described above, as one possible example. The ultrasound probe is a portable device which is manually placed against the skin of a subject. The imaging surface for example has a width and height each in the range 2 to 10cm.

Figure 5 shows a first way to form skin contact electrodes 210. As described above, each CMUT cell has a second (top) electrode. In the example shown in Figure 5, the skin contact electrodes 210 are formed from the same layer as the second electrodes.

The skin contact electrodes 210 are larger in area than the individual CMUT cells 204. In this example, each skin contact electrode is formed as a connected (and optionally enlarged) set of the second electrodes. The ultrasound functionality of those cells is sacrificed when the skin contact electrode is to be used, but the structure remains the same. This provides a fully integrated implementation of the skin contact electrodes 210.

In some designs, the functionality of the cells may still be used when operating in an imaging only mode. The top (second) electrodes of the CMUT cells may all connect to the ground electrode during ultrasound imaging. This is one option, as explained above. A switch in the ASIC may be used to provide the option of connecting the top electrodes to ground and use the probe in ultrasound mode, or else to switch the top electrodes to the sensing function to measure skin impedance. In this way, the ultrasound imaging functionality may be preserved, but not at the same time.

By way of example, the skin contact electrodes have a maximum linear dimension in the range 1mm to 1cm and the individual CMUT cells have a maximum linear dimension in the range 0.01 to 0.5 mm.

Note that a transducer may be considered to be the smallest individually addressable element. It may be formed of multiple cells (also known as drums) of the type described above. The dimension range above relates to the individual cells.

The CMUT cells may be integrated with an integrated circuit produced in a regular CMOS IC production process on large silicon wafers. The top electrodes for all of the CMUT cells are manufactured by first depositing a conducting material over the full area of all transducer elements and then selectively removing parts of this layer by an etching process through a mask, to leave only the individual electrodes on the individual membranes.

By simply adapting the etching mask, the conductive layer can be left intact over a larger area of membranes to form the larger skin contact electrodes 210.

These electrodes can be used for skin impedance measurement, in particular to determine the phasic, i.e. galvanic, skin response. They may additionally be used for ECG measurements.

In one set of examples, the CMUT cells (in particular the membranes) below the skin contact electrodes are not electronically connected to send and receive ultrasound waves so will only function as the mechanical substrate of the large electrodes.

In another set of example, the CMUT cells below the skin contact electrodes may be switchable between an imaging mode and a skin impedance measurement mode. Thus, the ASIC may be modified to enable this dual functionality of the CMUT cells at the locations of the skin contact electrodes.

The rest of the CMUT elements will receive an individual electrode to act as regular ultrasound transducer elements.

Figure 6a shows the structure of the device in a region where the individual CMUT cells 204 are formed.

A different design is shown compared to Figure 1.

A silicon membrane (i.e. the substrate) is shown as 300.

The CMUT membranes can be monolithically integrated on the ASIC. The ASIC itself is processed on a silicon wafer in a standard CMOS clean room. This ASIC wafer is then used as the substrate 300 to process the CMUT transducers directly on top in a MEMS clean room. This is a known processing sequence.

The top electrodes 120 are on top of the substrate, and a silicon dioxide insulation layer 302 is beneath. The vacuum cavity 118 is formed in the layer 302 and the bottom electrode 122 is beneath.

Figure 6b shows the structure of the same device as Figure 6A in a region where the skin electrode contacts 210 are formed. A larger single electrode is formed over a sub-array of the CMUT cells.

There may be just two skin electrode contacts, and they may be used sequentially for ECG and impedance measurement. There may instead be dedicated ECG skin contact electrodes and separate dedicated impedance measurement electrodes.

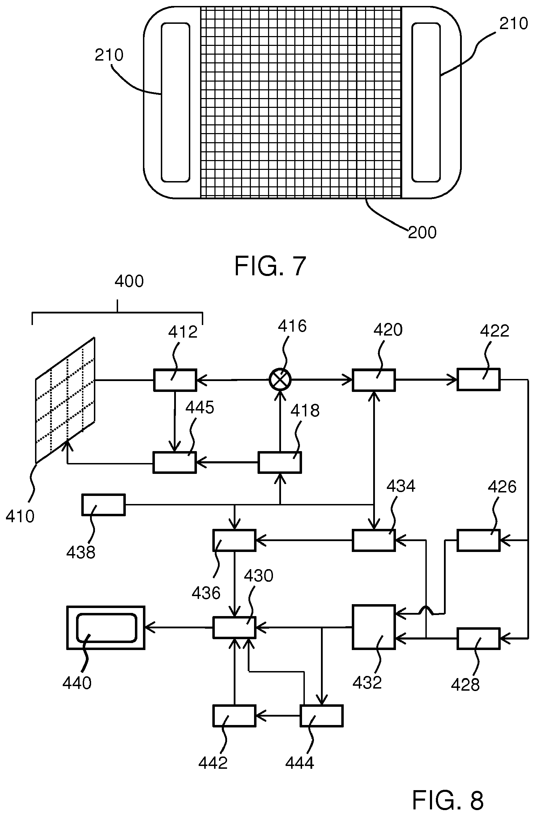

Figure 7 shows an alternative in which the skin contact electrodes 210 are formed on an area of the integrated circuit separate to the area occupied by the CMUT array 200, but still preferably using the same electrode layer that forms the top electrodes of the CMUT cells.

The skin contact electrodes areas can be covered by a protective conductive layer to protect them from moisture and other skin substances when placed in contact with the skin. The CMUT cell electrodes can be covered by an insulator layer.

As mentioned above, the invention enables a level of discomfort or pain to be assessed. It is well known that skin conductance changes when a person sweats, and that such sweating may be brought about either by physical effort or by the emotional state of a person.

An increase in skin conductance is the result of sweating that fills the sweat glands with salty sweat. Filled sweat glands form a conductance path to bloodstreams beneath, the latter having very high conductance. The skin conductance can be measured by placing electrodes on the skin, applying a voltage and measuring the current.

Sweating is partly caused by the body temperature regulation process, and partly by an effect that depends on the user's emotional state. Both effects take place at the same time, and they combine to result in an overall skin conductance. The so-called tonic skin response or skin conductance level (SCL) is a response which varies relatively slowly over time and depends both on temperature regulation and emotional conditions. The so-called phasic response or skin conductance response (SCR), or galvanic skin response (GSR) is a response which varies relatively quickly over time and depends on the emotional triggers.

The emotional state of a user is generally the result of emotional triggers that have happened in the recent past. These emotional triggers result in the more short term phasic variations in the skin conductance. These phasic responses are minimally dependent on body temperature related processes. However, they have relatively small amplitude and are easily disturbed.

A pure skin conductance measurement device cannot distinguish between the emotional component of the tonic response and the thermal regulation component. The measurement will see the effect of all conductance variations in parallel.

A skin conductance sensor typically applies a voltage (or current) to the skin, and measures a resulting response. This response may be a current flowing though (or a voltage across) the skin. In the latter case, voltage is applied to the skin through a series resistor, so that the skin and the series resistor function as a variable voltage divider. A current flowing is measured using a current amplifier and a voltage is sensed using a voltage amplifier.

The sensor may use a single ended design or a differential design. A single ended design is of lower cost and less sensitive to noise. A differential design is more complex but enables common mode effects to be cancelled. However, a sensor device which measures skin conductance needs to be able to provide separation of these two components using signal processing, if actions are to be taken in response to the emotional state alone.

The applicant has devised a sensor electrical circuit for the detection of skin responses. The sensor enables detection of the overall tonic (i.e. SCL) response and separation of the phasic (i.e. SCR) signals and over a wide range of skin conductance. The circuit operation is based on the recognition that the ratio of SCR and SCL amplitudes has a similar order of magnitude independent of the absolute skin conductance value.

The phasic skin response can be obtained by:
detecting local maxima and minima in a digital output signal representing the skin conductance;
detecting rising edges in the digital output signal representing the skin conductance; and
identifying as the phasic skin response those the rising edges that have a duration within a first range and an amplitude change within a second range.

Increases in the tonic skin response can be obtained by:
detecting rises in the digital output signal representing the skin conductance over a period at least longer than the maximum of the first range.

Thus, short duration rises in skin conductance are determined to be phasic responses whereas slower rises are determined to be caused by increases in tonic skin response.

Full details of this approach may be found in WO 2017/202620. It provides a low cost circuit for separating the phasic and tonic skin response signals. However, other known approaches may be used.

The controller is adapted in order to provide the function of skin impedance measurement and optionally also ECG measurement. All of the transducer (e.g. CMUT) cells, including the skin contact electrodes and cell electrodes, are connected to the ultrasound system by means of an ASIC. In known systems the ASIC drives only the ultrasound devices. The ASIC can however be adapted to carry out more driving tasks than just the ultrasound imaging.

For skin impedance measurement, from which the phasic GSR measurement is derived, the ASIC is adapted to apply a low voltage (typically 0.1V - 3V) between the electrodes and measure the current to assess the skin impedance. As the corresponding skin conductance is typically in the order of microSiemens, it is advantageous to incorporate a current amplifier with a very high input impedance in the ASIC design.

For ECG measurement, a low input impedance voltage amplifier is incorporated in the ASIC to amplify and measure the voltages of the electrodes for ECG measurement, when placed in contact with the skin. The ASIC may incorporate either the current amplifier, or the voltage amplifier or both.

The ultrasound signals and skin electrode signals can be transported either wirelessly or over a cable (e.g. USB) to a PC, tablet or mobile phone to record and show the ultrasound, GSR, and/or ECG simultaneously. The information may be interpreted by either human operator or by means of a machine learning or AI based algorithms. The probe can also be used independently as only an ultrasound probe, a GSR probe, or an ECG probe, on different parts of the body.

For completeness, Figure 8 shows an ultrasonic diagnostic imaging system with an array transducer probe 400 in block diagram form, and is used to explain the general operation of an exemplary ultrasound imaging system.

The system comprises an array transducer probe 400 which has a transducer array 410 for transmitting ultrasound waves and receiving echo information. The transducer array 410 may comprise CMUT transducers; piezoelectric transducers, formed of materials such as PZT or PVDF; or any other suitable transducer technology. In this example, the transducer array 410 is a two-dimensional array of CMUT transducers capable of scanning either a 2D plane or a three dimensional volume of a region of interest. In another example, the transducer array may be a 1D array.

The transducer array 410 is coupled to a microbeamformer 412 which controls reception of signals by the transducer elements. Microbeamformers are capable of at least partial beamforming of the signals received by sub-arrays, generally referred to as "groups" or "patches", of transducers as described in US Patents 5,997,479 (Savord et al.), 6,013,032 (Savord), and 6,623,432 (Powers et al.).

It should be noted that the microbeamformer is entirely optional. Further, the system includes a transmit/receive (T/R) switch 416, which the microbeamformer 412 can be coupled to and which switches the array between transmission and reception modes, and protects the main beamformer 420 from high energy transmit signals in the case where a microbeamformer is not used and the transducer array is operated directly by the main system beamformer. The transmission of ultrasound beams from the transducer array 410 is directed by a transducer controller 418 coupled to the microbeamformer by the T/R switch 416 and a main transmission beamformer (not shown), which can receive input from the user's operation of the user interface or control panel 438. The controller 418 can include transmission circuitry arranged to drive the transducer elements of the array 410 (either directly or via a microbeamformer) during the transmission mode.

In a typical line-by-line imaging sequence, the beamforming system within the probe may operate as follows. During transmission, the beamformer (which may be the microbeamformer or the main system beamformer depending upon the implementation) activates the transducer array, or a sub-aperture of the transducer array. The sub-aperture may be a one dimensional line of transducers or a two dimensional patch of transducers within the larger array. In transmit mode, the focusing and steering of the ultrasound beam generated by the array, or a sub-aperture of the array, are controlled as described below.

Upon receiving the backscattered echo signals from the subject, the received signals undergo receive beamforming (as described below), in order to align the received signals, and, in the case where a sub-aperture is being used, the sub-aperture is then shifted, for example by one transducer element. The shifted sub-aperture is then activated and the process repeated until all of the transducer elements of the transducer array have been activated.

For each line (or sub-aperture), the total received signal, used to form an associated line of the final ultrasound image, will be a sum of the voltage signals measured by the transducer elements of the given sub-aperture during the receive period. The resulting line signals, following the beamforming process below, are typically referred to as radio frequency (RF) data. Each line signal (RF data set) generated by the various sub-apertures then undergoes additional processing to generate the lines of the final ultrasound image. The change in amplitude of the line signal with time will contribute to the change in brightness of the ultrasound image with depth, wherein a high amplitude peak will correspond to a bright pixel (or collection of pixels) in the final image. A peak appearing near the beginning of the line signal will represent an echo from a shallow structure, whereas peaks appearing progressively later in the line signal will represent echoes from structures at increasing depths within the subject.

One of the functions controlled by the transducer controller 418 is the direction in which beams are steered and focused. Beams may be steered straight ahead from (orthogonal to) the transducer array, or at different angles for a wider field of view. The steering and focusing of the transmit beam may be controlled as a function of transducer element actuation time.

Two methods can be distinguished in general ultrasound data acquisition: plane wave imaging and "beam steered" imaging. The two methods are distinguished by a presence of the beamforming in the transmission ("beam steered" imaging) and/or reception modes (plane wave imaging and "beam steered" imaging).

Looking first to the focusing function, by activating all of the transducer elements at the same time, the transducer array generates a plane wave that diverges as it travels through the subject. In this case, the beam of ultrasonic waves remains unfocused. By introducing a position dependent time delay to the activation of the transducers, it is possible to cause the wave front of the beam to converge at a desired point, referred to as the focal zone. The focal zone is defined as the point at which the lateral beam width is less than half the transmit beam width. In this way, the lateral resolution of the final ultrasound image is improved.

For example, if the time delay causes the transducer elements to activate in a series, beginning with the outermost elements and finishing at the central element(s) of the transducer array, a focal zone would be formed at a given distance away from the probe, in line with the central element(s). The distance of the focal zone from the probe will vary depending on the time delay between each subsequent round of transducer element activations. After the beam passes the focal zone, it will begin to diverge, forming the far field imaging region. It should be noted that for focal zones located close to the transducer array, the ultrasound beam will diverge quickly in the far field leading to beam width artifacts in the final image. Typically, the near field, located between the transducer array and the focal zone, shows little detail due to the large overlap in ultrasound beams. Thus, varying the location of the focal zone can lead to significant changes in the quality of the final image.

It should be noted that, in transmit mode, only one focus may be defined unless the ultrasound image is divided into multiple focal zones (each of which may have a different transmit focus).

In addition, upon receiving the echo signals from within the subject, it is possible to perform the inverse of the above described process in order to perform receive focusing. In other words, the incoming signals may be received by the transducer elements and subject to an electronic time delay before being passed into the system for signal processing. The simplest example of this is referred to as delay-and-sum beamforming. It is possible to dynamically adjust the receive focusing of the transducer array as a function of time.

Looking now to the function of beam steering, through the correct application of time delays to the transducer elements it is possible to impart a desired angle on the ultrasound beam as it leaves the transducer array. For example, by activating a transducer on a first side of the transducer array followed by the remaining transducers in a sequence ending at the opposite side of the array, the wave front of the beam will be angled toward the second side. The size of the steering angle relative to the normal of the transducer array is dependent on the size of the time delay between subsequent transducer element activations.

Further, it is possible to focus a steered beam, wherein the total time delay applied to each transducer element is a sum of both the focusing and steering time delays. In this case, the transducer array is referred to as a phased array.

In case of the CMUT transducers, which require a DC bias voltage for their activation, the transducer controller 418 can be coupled to control a DC bias control 445 for the transducer array. The DC bias control 445 sets DC bias voltage(s) that are applied to the CMUT transducer elements.

For each transducer element of the transducer array, analog ultrasound signals, typically referred to as channel data, enter the system by way of the reception channel. In the reception channel, partially beamformed signals are produced from the channel data by the microbeamformer 412 and are then passed to a main receive beamformer 420 where the partially beamformed signals from individual patches of transducers are combined into a fully beamformed signal, referred to as radio frequency (RF) data. The beamforming performed at each stage may be carried out as described above, or may include additional functions. For example, the main beamformer 420 may have 128 channels, each of which receives a partially beamformed signal from a patch of dozens or hundreds of transducer elements. In this way, the signals received by thousands of transducers of a transducer array can contribute efficiently to a single beamformed signal.

The beamformed reception signals are coupled to a signal processor 422. The signal processor 422 can process the received echo signals in various ways, such as: band-pass filtering; decimation; I and Q component separation; and harmonic signal separation, which acts to separate linear and nonlinear signals so as to enable the identification of nonlinear (higher harmonics of the fundamental frequency) echo signals returned from tissue and microbubbles. The signal processor may also perform additional signal enhancement such as speckle reduction, signal compounding, and noise elimination. The band-pass filter in the signal processor can be a tracking filter, with its pass band sliding from a higher frequency band to a lower frequency band as echo signals are received from increasing depths, thereby rejecting noise at higher frequencies from greater depths that is typically devoid of anatomical information.

The beamformers for transmission and for reception are implemented in different hardware and can have different functions. Of course, the receiver beamformer is designed to take into account the characteristics of the transmission beamformer. In Figure 8 only the receiver beamformers 412, 420 are shown, for simplicity. In the complete system, there will also be a transmission chain with a transmission micro beamformer, and a main transmission beamformer.

The function of the micro beamformer 412 is to provide an initial combination of signals in order to decrease the number of analog signal paths. This is typically performed in the analog domain.

The final beamforming is done in the main beamformer 420 and is typically after digitization.

The transmission and reception channels use the same transducer array 410 which has a fixed frequency band. However, the bandwidth that the transmission pulses occupy can vary depending on the transmission beamforming used. The reception channel can capture the whole transducer bandwidth (which is the classic approach) or, by using bandpass processing, it can extract only the bandwidth that contains the desired information (e.g. the harmonics of the main harmonic).

The RF signals may then be coupled to a B mode (i.e. brightness mode, or 2D imaging mode) processor 426 and a Doppler processor 428. The B mode processor 426 performs amplitude detection on the received ultrasound signal for the imaging of structures in the body, such as organ tissue and blood vessels. In the case of line-by-line imaging, each line (beam) is represented by an associated RF signal, the amplitude of which is used to generate a brightness value to be assigned to a pixel in the B mode image. The exact location of the pixel within the image is determined by the location of the associated amplitude measurement along the RF signal and the line (beam) number of the RF signal. B mode images of such structures may be formed in the harmonic or fundamental image mode, or a combination of both as described in US Pat. 6,283,919 (Roundhill et al.) and US Pat. 6,458,083 (Jago et al.) The Doppler processor 428 processes temporally distinct signals arising from tissue movement and blood flow for the detection of moving substances, such as the flow of blood cells in the image field. The Doppler processor 428 typically includes a wall filter with parameters set to pass or reject echoes returned from selected types of materials in the body.

The structural and motion signals produced by the B mode and Doppler processors are coupled to a scan converter 432 and a multi-planar reformatter 444. The scan converter 432 arranges the echo signals in the spatial relationship from which they were received in a desired image format. In other words, the scan converter acts to convert the RF data from a cylindrical coordinate system to a Cartesian coordinate system appropriate for displaying an ultrasound image on an image display 440. In the case of B mode imaging, the brightness of pixel at a given coordinate is proportional to the amplitude of the RF signal received from that location. For instance, the scan converter may arrange the echo signal into a two dimensional (2D) sector-shaped format, or a pyramidal three dimensional (3D) image. The scan converter can overlay a B mode structural image with colors corresponding to motion at points in the image field, where the Doppler-estimated velocities to produce a given color. The combined B mode structural image and color Doppler image depicts the motion of tissue and blood flow within the structural image field. The multi-planar reformatter will convert echoes that are received from points in a common plane in a volumetric region of the body into an ultrasound image of that plane, as described in US Pat. 6,443,896 (Detmer). A volume renderer 442 converts the echo signals of a 3D data set into a projected 3D image as viewed from a given reference point as described in US Pat. 6,530,885 (Entrekin et al.).

The 2D or 3D images are coupled from the scan converter 32, multi-planar reformatter 444, and volume renderer 442 to an image processor 430 for further enhancement, buffering and temporary storage for display on an image display 440. The imaging processor may be adapted to remove certain imaging artifacts from the final ultrasound image, such as: acoustic shadowing, for example caused by a strong attenuator or refraction; posterior enhancement, for example caused by a weak attenuator; reverberation artifacts, for example where highly reflective tissue interfaces are located in close proximity; and so on. In addition, the image processor may be adapted to handle certain speckle reduction functions, in order to improve the contrast of the final ultrasound image.

In addition to being used for imaging, the blood flow values produced by the Doppler processor 428 and tissue structure information produced by the B mode processor 426 are coupled to a quantification processor 434. The quantification processor produces measures of different flow conditions such as the volume rate of blood flow in addition to structural measurements such as the sizes of organs and gestational age. The quantification processor may receive input from the user control panel 438, such as the point in the anatomy of an image where a measurement is to be made.

Output data from the quantification processor is coupled to a graphics processor 436 for the reproduction of measurement graphics and values with the image on the display 440, and for audio output from the display device 440. The graphics processor 436 can also generate graphic overlays for display with the ultrasound images. These graphic overlays can contain standard identifying information such as patient name, date and time of the image, imaging parameters, and the like. For these purposes the graphics processor receives input from the user interface 438, such as patient name. The user interface is also coupled to the transmit controller 418 to control the generation of ultrasound signals from the transducer array 410 and hence the images produced by the transducer array and the ultrasound system. The transmit control function of the controller 418 is only one of the functions performed. The controller 418 also takes account of the mode of operation (given by the user) and the corresponding required transmitter configuration and band-pass configuration in the receiver analog to digital converter. The controller 418 can be a state machine with fixed states.

The user interface is also coupled to the multi-planar reformatter 444 for selection and control of the planes of multiple multi-planar reformatted (MPR) images which may be used to perform quantified measures in the image field of the MPR images.

The controller 418 may function as the controller for performing the skin impedance and/or ECG measurements, by using the signals generated by the ASIC in the CMUT probe. Thus, the overall funciton of the controller as dicussed above may be divided between the CMUT probe and the controller in the remote unit to which the probe is connected (wired or wirelessly).

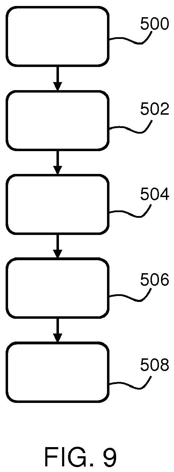

Figure 9 shows an imaging method comprising:
in step 500, applying a sensor head to an external surface of a body for imaging tissue beneath the surface, wherein the sensor head comprises an ultrasound transducer array and at least one pair of skin contact electrodes;
in step 502, controlling the ultrasound transducer array to generate ultrasound image data;
in step 504, performing skin impedance measurement using the skin contact electrodes; and
in step 506, analyzing the skin impedance over time to determine a pain or discomfort level of the subject.

The method additionally optionally comprises performing ECG measurements using the skin contact electrodes in step 508.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. An imaging system comprising:
a sensor head (400) for application to an external surface of a body for imaging tissue beneath the surface, wherein the sensor head comprises:
an ultrasound transducer array (200); and
at least one pair of skin contact electrodes (210); and
a controller (418), which is adapted to:
control the ultrasound transducer array to generate ultrasound image data;
perform skin impedance measurement using the skin contact electrodes; and
analyze the skin impedance over time to determine a pain or discomfort level of the subject.

2. A system as claimed in claim in claim 1, wherein the controller (418) is adapted to determine a phasic skin response.

3. A system as claimed in claim 1 or 2, wherein the controller (418) is further adapted to perform ECG measurements using the skin contact electrodes.

4. A system as claimed in any one of claims 1 to 3, wherein the ultrasound array is formed on a substrate (300) and the skin contact electrodes are formed on the same substrate.

5. A system as claimed in any one of claims 1 to 4, wherein the ultrasound transducer array comprises a capacitive micromachined ultrasound transducer, CMUT, array.

6. A system as claimed in claim 5, wherein each transducer of the CMUT array comprises:
a first electrode (122) connected to the substrate;
a flexible membrane (114), wherein the flexible membrane is at least partially spatially separated from the first electrode; and
a second electrode (120) connected to the flexible membrane,

7. A system as claimed in claim 6, wherein the controller comprises drive electronics having:
a first output for delivering a DC bias voltage to first electrodes of the CMUT transducers;
a second output for delivering an AC drive signal to the second electrodes of the CMUT transducers; and
a capacitance sensing circuit.

8. A system as claimed in claim 6 or 7, wherein the skin contact electrodes (210) are formed from the same layer as the second electrode (120).

9. A system as claimed in claim 7, wherein the skin contact electrodes (210) are larger in area than the individual cells of the transducers of the transducer array, for example wherein the skin contact electrodes have a maximum linear dimension in the range 1mm to 1cm and individual CMUT transducer cells of the transducers of the transducer array have a maximum linear dimension in the range 0.01 to 0.5 mm.

10. A system as claimed in claim 9, wherein the skin contact electrodes (210) each comprise a connected set of second electrodes of a sub-array of the CMUT transducers.

11. A system as claimed in any one of claims 1 to 10, wherein the controller comprises an application specific integrated circuit, ASIC, wherein the transducers of the transducer array are integrated with the ASIC.

12. A system as claimed in claim 11, wherein the ASIC comprises a signal amplifier for the signals received by the skin contact electrodes.

13. A system as claimed in any one of claims 1 to 12, further comprising a wireless transmitter for transmitting ultrasound data and data from the skin contact electrodes to a remote device for display and/or processing.

14. A system as claimed in any one of claims 1 to 13 which is controllable independently as:
an ultrasound probe;
a skin impedance analysis system, and
an ECG recordal system.

15. An imaging method comprising:
applying a sensor head to an external surface of a body for imaging tissue beneath the surface, wherein the sensor head comprises an ultrasound transducer array and at least one pair of skin contact electrodes;
controlling the ultrasound transducer array to generate ultrasound image data;
performing skin impedance measurement using the skin contact electrodes; and
analyzing the skin impedance over time to determine a pain or discomfort level of the subject,
and optionally performing ECG measurements using the skin contact electrodes.
